Europäisches Patentamt

European Patent Office

Office européen des brevets

(19)

(11) Numéro de publication: **0 123 637**
**B1**

(12) **FASCICULE DE BREVET EUROPÉEN**

(45) Date de publication du fascicule du brevet:
**25.11.87**

(51) Int. Cl.⁴: **A 61 K 7/48**, A 61 K 9/06

(21) Numéro de dépôt: **84430013.7**

(22) Date de dépôt: **25.04.84**

(54) **Pommade dermique et dermatologique à large spectre d'activité, et son procédé de préparation.**

(30) Priorité: **26.04.83 FR 8306990**

(43) Date de publication de la demande:
**31.10.84 Bulletin 84/44**

(45) Mention de la délivrance du brevet:
**25.11.87 Bulletin 87/48**

(84) Etats contractants désignés:
**AT BE CH DE FR GB IT LI LU NL SE**

(56) Documents cité:
**CA-A-1 015 664**
**CH-A-272 709**
**FR-A-661 398**
**FR-A-855 537**
**FR-A-2 260 992**

(73) Titulaire: **Soto, Lucien, Résidence "Le Loubassane" - Bât. S Avenue du Docteur Bertrand, F-13090 Aix- en- Provence (FR)**

(72) Inventeur: **Soto, Lucien, Résidence "Le Loubassane" - Bât. S Avenue du Docteur Bertrand, F-13090 Aix- en- Provence (FR)**

(74) Mandataire: **Marek, Pierre, 28 & 32 rue de la Loge, F-13002 Marseille (FR)**

EP 0 123 637 B1

LIBER, STOCKHOLM 1987

## Description

La présente invention concerne une pommade dermique et dermatologique à large spectre d'activité contenant comme ingrédients actifs, de l'huile d'olive et de la cire d'abeille. Elle vise aussi le procédé de préparation de cette pommade.

L'action de la pommade selon l'invention est notamment efficace: dans le traitement des dermites et dermatoses d'origine diverse: infectieuse, parasitaire, tumorale nerveuse, allergique professionnelle (gale du ciment); dans le traitement des brûlures par contact (contact de la peau avec des liquides, des objets, des vapeurs, des gaz brûlants, des matières incandescentes,) ou par rayonnement (coup de soleil, radiations de nature diverse); dans le traitement des engelures et des gerçures; dans le traitement des plaies superficielles ou profondes; dans le traitement des dermatomycoses; dans le traitement des prurits localisés; dans le traitement des piqûres d'insectes, dans la résorption des hématomes. En outre, la pommade selon l'invention peut être avantageusement utilisée à des fins hygiéniques ou prophylactiques, ou cosmétiques, ou comme produit de soins corporels ou de beauté, ou comme onguent ou crème de protection contre le rayonnement solaire.

Cette pommade possède notamment des activités anti-inflammatoires, antiseptiques, anesthésiques, antimycosiques, régénératives, cicatrisantes.

On connaît (CA-A-1 015 664), un onguent pour le traitement du psoriasis, contenant de l'huile d'olive et de la cire d'abeille, mais ces substances entrent dans la composition dudit onguent, seulement comme excipients inertes, et dans des proportions très inférieures à celles des ingrédients actifs.

On connaît aussi (FR-A-2 260 992), un onguent pour le traitement des brûlures, contenant, entre autres principes actifs, de l'huile d'olive vierge et de la cire blanche d'abeille. Toutefois, le spectre d'activité de cet onguent est restreint et on observe que les principaux principes actifs entrant dans sa composition sont, du moins sur le plan quantitatif, l'huile d'olive, la résine de pin et le suif de mouton, tandis que la cire d'abeille est une cire blanche, c'est-à-dire une cire raffinée.

La pommade ou onguent dermique et dermatologique selon l'invention est plus particulièrement remarquable par le fait que l'huile d'olive et la cire d'abeille entrant dans sa composition sont, respectivement, de l'huile d'olive vierge et de la cire d'abeille vierge, et en ce que ces substances constituent les deux seuls ou les deux ingrédients actifs principaux de ladite pommade, notamment sur le plan quantitatif.

Selon une autre disposition caractéristique, la pommade selon l'invention contient également, comme substance pharmacologiquent active, une ou plusieurs essences de plantes aromatiques, telles qu'essences de lavande, de lavandin, de thym, de genièvre et/ou de genévrier, par exemple et de préférence, dans la proportion de 1 partie d'essence(s) de plante(s) aromatique(s) pour 100 parties d'huile d'olive vierge et 10 parties de cire d'abeille vierge.

Suivant le procédé de l'invention, on procède d'abord au mélange de l'huile d'olive vierge et de la cire d'abeille vierge, à l'aide de tous matériels appropriés connus en soi.

Par exemple, pour l'obtention de un kilogramme de pommade environ, on mélange 1 litre d'huile d'olive vierge et 100 grammes de cire d'abeille vierge.

L'huile d'olive vierge est l'huile obtenue par la première pression à froid des fruits de l'olivier, et n'ayant subi aucun traitement complémentaire, ni adjonction.

La cire d'abeille vierge est la cire extraite des gâteaux de cire ou de miel retirés de la ruche et n'ayant subi aucun traitement. Les gâteaux de cire extraits des rayons de la ruche sont simplement fondus et coulés dans des moules ou récipients (pour des raisons pratiques de stockage et de manipulation, ou de commercialisation). La cire est seulement filtrée, lors de son coulage dans ces moules, au moyen de tamis très fins (métalliques ou en soie), afin de retenir les éléments et impuretés indésirables (abdomens d'abeilles, etc.). Dans la mixture selon l'invention, la cire d'abeille vierge est donc utilisée sous son état naturel, sans modification de cet état, et sans adjonction d'aucun produit chimique quel qu'il soit. Elle se présente sous forme d'une substance molle et jaunâtre.

On souligne le fait que plus l'huile d'olive et la cire d'abeille sont vierges, meilleures sont les vertus de la pommade obtenue.

L'huile d'olive vierge et la cire d'abeille vierge placées dans un récipient adéquat, éventuellement équipé d'un dispositif d'agitation ou d'un mélangeur approprié, sont chauffées, au bain-marie, de manière à porter lentement le mélange obtenu à une température de 80-100°C, tout en agitant continuellement ledit mélange de façon à obtenir une mixtion parfaite de l'huile d'olive et de la cire d'abeille.

Lorsque le mélange est parfaitement homogène, on arrête le chauffage et, pendant que ce mélange se trouve encore à l'état liquide, on ajoute 1 cl d'une essence ou de plusieurs essences de plantes aromatiques (essence de lavande, lavandin, romarin, thym, genièvre et genévrier) tout en remuant.

Avant refroidissement et avant que la mixtion ne prenne une consistance pâteuse, on la verse dans les récipients ou pots d'utilisation et on laisse ensuite refroidir à la température ambiante.

La pommade ainsi obtenue se conserve facilement, de préférence dans un endroit frais, sans limite de durée de conservation et elle garde indéfiniment toutes ses propriétés et toute son efficacité.

La pommade dermique et dermatologique selon la présente invention a été utilisée efficacement pour le traitement de brûlures, de dermatoses, de dermites, de mycoses, etc.

On indique, ci-après, quelques exemples de traitements thérapeutiques ayant démontré l'activité pharmacologique efficace de cette pommade.

### Dermatose professionnelle dite "gale du ciment"

La pommade a été étendue en applications bi-quotidiennes sur les zones prurigineuses de la peau d'une centaine de personnes adultes, de sexe masculin, atteintes de gale du ciment. 48 heures après cette application, les papules et les démangeausons avaient disparu.

### Dermatomycoses

La pommade a èté étendue en applications quotidiennes ou bi-quotidiennes sur le tissu épidermique malade de plusieurs personnes adultes, de sexe féminin et masculin, atteintes de dermatomycoses non identifiées. Après 4 jours d'application de la pommade, toute trace de champignons sur la peau avait disparu.

### Brûlures

La pommade a été appliquée, une ou deux fois par jour, sur les lésions résultant de brûlures superficielles et profondes de plusieurs dizaines d'enfants et d'adultes, de sexe masculin et féminin souffrant de brûlures par contact ou par radiation (coups de soleil). Immédiatement après application de la pommade sur les lésions, on a constaté une atténuation de la douleur et une stabilisation de ces dernières. D'autre part, on a observé une accélération de la régénération des tissus atteints et de la cicatrisation.

Lors des différents traitements basés sur une application de la pommade selon l'invention, on n'a constaté aucun cas de réaction d'allergie à ladite pommade.

Suivant la nature des troubles ou atteintes dermatologiques ou du traitement dermique, la pommade selon l'invention s'emploie en application locale directe, c'est-à-dire qu'elle est étalée en couche mince sur la région prurigineuse ou sur les lésions, ou elle est étalée préalablement sur une gaze stérile qui est ensuite posée en recouvrement de la zone affectée et maintenue en place par un pansement adéquat.

Suivant la nature du trouble ou de l'affection de la peau, la pommade peut être aussi appliquée par friction sur la région malade, afin de favoriser sa pénétration dans le tissu épidermique et/ou dermique.

La fréquence des applications de cette pommade est de 1 à 3 fois par jour.

### Revendications

1. Pommade dermique et dermatologique à large spectre d'activité contenant, comme ingrédients actifs, de l'huile d'olive et de la cire d'abeille, càractérisée en ce que l'huile d'olive et la cire d'abeille entrant dans sa composition sont, respectivement, de l'huile d'olive vierge et de la cire d'abeille vierge, dans la proportion de 100 parties d'huile d'olive vierge pour 10 parties de cire d'abeille vierge, et en ce que ces substances constituent les deux seuls ou les deux ingrédients actifs principaux de ladite pommade, sur le plan quantitatif.

2. Pommade dermique et dermatologique suivant la revendication1, caractérisée en ce qu'elle contient également comme ingrédient(s) actif(s) une ou plusieurs essences de plantes aromatiques telles que essence de lavande, de lavandin, de romarin, de thym, de genièvre, de genévrier.

3. Pommade selon la revendication 2, caractérisée en ce que l'essence ou les essences de plantes aromatiques est ou sont contenue(s) dans ladite pommade dans une proportion de 1 partie d'essence(s) de plante(s) aromatique(s) pour 100 parties d'huile d'olive vierge et 10 parties de cire d'abeille vierge.

4. Procédé de fabrication de la pommade dermique ou dermatologique suivant l'une des revendications 2 ou 3, caractérisé en ce que l'on procède d'abord au mélange de l'huile d'olive vierge et de la cire d'abeille vierge, en les chauffant lentement, au bain-marie, tout en agitant, lusqu'à ce que le mélange homogène obtenu solt porté a une température de 80°-100° C; on arrête ensuite le chauffage et, pendant que le mélange se trouve encore à l'état liquide, on ajoute l'essence ou les essences de plante aromatiques telles qu'essence de lavande, de lavandin, de romarin, de thym, de genièvre, de genévrier, tout en remuant; enfin, avant que la mixtion ne prenne une consistance pâteuse, on la verse dans les récipients ou pots d'utilisation dans lesquels on la laisse refroidir.

5. Pommade dermatologique suivant l'une quelconque des revendications 1 à 3, ou obtenue selon le procédé de la revendication 4, caractérisée par son utilisation thérapeutique pour le traitement des troubles, affections et lésions dermatologiques.

6. Pommade dermatologique selon la revendication 5, caractérisée par son utilisation pour le traitement des dermitoses, des dermites, des prurits localisés, des dermatomycoses, des piqûres d'insectes.

7. Pommade dermatologique suivant la revendication 5, caractérisée per son utilisation pour le traitement des brûlures.

8. Pommade dermatologique selon la revendication 5, caractérisé par son utilisation dans le traitement des plaies superficielles ou profondes, des engelures, des gerçures, des hématomes.

9. Pommade dermique suivant l'une quelconque des revendications 1 à 3, ou obtenue suivant le procédé de la revendication 4, caractérisée par son utilisation comme produit d'hygiène, de prophylaxie ou de soins corporels, ou comme produit de beauté, ou comme onguent de protection contre les radiations solaires.

## Patentansprüche

1. Dermatologische Salbe mit Breitspektrums-Aktivität, welche als aktive Bestandteile Olivenöl und Bienenwachs enthält, dadurch gekennzeichnet, daß das an der Zusammensetzung beteiligte Olivenöl und das Bienenwachs aus erstgepreßtem Olivenöl bzw. reinem Bienenwachs im Verhältnis von 100 Teilen erstgepreßtem Olivenöl zu 10 Teilen reinem Bienenwachs bestehen, und daß diese Substanzen mengenmäßig die beiden einzigen oder die beiden aktiven Häuptbestandteile der besagten Salbe bilden.

2. Dermatologische Salbe nach Anspruch 1, dadurch gekennzeichnet, daß sie auch als aktiven Bestandteil(e) einen oder mehrere Extrakte aus Aromapflanzen wie zum Beispiel Lavendel-, Lavendelhybriden-, Rosmarin-, Thymian-, Wacholderbeeren-, Wacholderstrauchextrakt enthält.

3. Salbe nach Anspruch 2, dadurch gekennzeichnet, daß der Extrakt oder die Extrakte aus Aromapflanzen in der besagten Salbe in Verhältnis von 1 Teil Aromapflanzenextrakt(en) zu 100 Teilen erstgepreßtem Olivienöl und 10 Teilen reinem Bienenwachs enthalten ist oder sind.

4. Verfahren zur Herstellung der dermatologischen Salbe nach einem der Ansprüche 2 oder 3, dadurch gekennzeichnet, daß man zuerst die Mischung des erstgepreßten Olivenöls mit dem reinen Bienenwachs vornimmt, in dem man sie im Wasserbad unter Schütteln langsam erwärmt, bis die erzielte, homogene Mischung auf eine Temperatur von 80°-100° C gebracht ist; man die Erwärmung dann einstellt und, während die Mischung sich noch im flüssigen Zustand befindet, man den Extrakt oder die Extrakte aus Aromapflanzen wie zum Beispiel Lavendel-, Lavendelhybriden-, Rosmarin-, Thymian-, Wacholderbeeren-, Wacholderstrauchextrakt unter Rühren hinzufügt; und man sie schließlich bevor die Mischung eine dickflüssige Konsistenz annimmt, in Behälter oder Gebrauchstöpfchen gießt, worin man sie abkühlen läßt.

5. Dermatologische Salbe nach einem der Ansprüche 1 bis 3, oder gewonnen nach dem Verfahren nach Anspruch 4, gekennzeichnet durch ihre therapeutische Anwendung für die Behandlung von Hautstörungen, -leiden und -verletzungen.

6. Dermatologische Salbe nach Anspruch 5, gekennzeichnet durch ihre Anwendung für die Behandlung von Dermatosen, Dermiten, lokalem Juckreiz, Dermatomykosen und Insektenstichen.

7. Dermatologische Salbe nach Anspruch 5, gekennzeichnet durch ihre Anwendung für die Behandlung von Verbrennungen.

8. Dermatologische Salbe nach Anspruch 5, gekennzeichnet durch ihre Anwendung bei der Behandlung von oberflächlichen oder tiefen Wunden, Frostbeulen, Rissen und Hämatomen.

9. Dermatologische Salbe nach einem der Ansprüche 1 bis 3, oder gewonnen nach dem Verfahren nach Anspruch 4, gekennzeichnet durch ihre Verwendung als Hygiene-, Prophylaxe- oder Körperpflege-, oder als Kosmetikprodukt, oder als Schutzsalbe gegen Sonnenbestrahlung.

## Claims

1. A dermic and dermatological ointment with a broad spectrum of activity and containing, as active ingredients, olive oil and beeswax, characterised in that the olive oil and the beeswax of which it is composed are, respectively, virgin olive oil and virgin beeswax, in proportions of 100 parts of virgin olive oil to 10 parts of virgin beeswax, and in that these substances constitute the only two or the two main active ingredients of said ointment on a quantitative basis.

2. A dermic and dermatological ointment according to claim 1, characterised in that it also contains as active ingredient(s) one or more aromatic plant oils such as oil of lavender, of lavandin, of rosemary, of thyme, of juniper berries or of juniper.

3. An ointment according to claim 2, characterised in that the aromatic plant oil or oils is or are contained in said ointment in a proportion of 1 part of aromatic plant oil(s) to 100 parts of virgin olive oil and 10 parts of virgin beeswax.

4. A process for manufacturing the dermic or dermatological ointment according to claim 2 or claim 3, characterised in that firstly the virgin olive oil and the virgin beeswax are mixed by being heated slowly, in a water bath, and simultaneously stirred, until the homogeneous mixture obtained is brought to a temperature of 80°- 100°C; heating is then stopped and, while the mixture is still in the liquid state, the aromatic plant oil or oils such as oil of lavender, of lavandin, of rosemary, of thyme, of juniper berries or of juniper is or are added, with stirring; finally, before the mixture takes on a pasty consistency, it is poured into the containers or jars used, where it is left to cool.

5. A dermatological ointment according to any one of claims 1 to 3, or obtained by the process according to claim 4, characterised by its therapeutic use for the treatment of dermatological disorders, ailments and lesions.

6. A dermatological ointment according to claim 5, characterised by its use for the treatment of dermatosis, dermatitis, localized pruritus, dermatomycosis and insect bites.

7. A dermatological ointment according to claim 5, characterised by its use for the treatment of burns.

8. A dermatological ointment according to claim 5, characterised by its use in the treatment of superficial or deep wounds, chilblains, cracks or haemotomas.

9. A dermic ointment according to any one of claims 1 to 3, or obtained by the process according to claim 4, characterized by its use as a hygiene product, a disease prevention product or a product for care of the body, or as a beauty product, or as an ointment for protection against the sun's rays.